# EUROPÄISCHE PATENTSCHRIFT

(11) **EP 0 659 391 B1**
(45) Veröffentlichungstag und Bekanntmachung des Hinweises auf die Patenterteilung: **18.02.1998**
(21) Anmeldenummer: 94118113.3
(22) Anmeldetag: 17.11.1994
(51) Int. Cl.: A61F 15/00

(54) **Vorrichtung zur Portionierung von bandförmigem Material**
Tape portioning dispenser
Distributeur pour préportionner du ruban

(30) Priorität: 19.11.1993 US 155454
(43) Veröffentlichungstag der Anmeldung: 28.06.1995
(73) Patentinhaber: Holtsch, Maria ehemals Holtsch Metallwarenherstellung, 65232 Taunusstein-Wingsbach (DE)
(72) Erfinder: Holtsch, Peter, D-65232 Taunusstein-Wingsbach (DE)
(74) Vertreter: Eisenführ, Speiser & Partner

(56) Entgegenhaltungen:
- FR-A- 2 541 247
- FR-A- 2 581 915
- FR-A- 2 590 829
- FR-A- 2 606 000

## Beschreibung

Die Erfindung betrifft eine Vorrichtung zur Portionierung von bandförmigem Material, insbesondere Wund- oder Heftpflaster, mit einer Einrichtung zum Vorschub des bandförmigen Materials von einer Bandaufnahmeeinrichtung, einer auslenkbaren Bandschneideeinrichtung und einer Klemmeinrichtung, die das bandförmige Material bei Auslenkung der Bandschneideeinrichtung im wesentlichen fixiert.

Eine solche Vorrichtung ist aus dem dem deutschen Gebrauchsmuster G 92 00 572.1 bekannt.

Mit der bekannten Vorrichtung konnte zwar ein weitestgehend zufriedenes Ergebnis beim Portionieren von Wund- oder Heftpflaster erreicht werden, jedoch haben sich einige Schwächen gezeigt, die zu Fehlfunktionen oder Fehlbedienungen führten.

Bei der bekannten Einrichtung wird das bandförmige Material von der Vorschubeinrichtung durch einen Ausgabeschlitz befördert. Der Schlitz liegt in einer waagerechten Ebene, oberhalb der ein Messer der Bandschneideeinrichtung liegt. Wird die Bandschneideeinrichtung entlang einer Führung ausgelenkt, wird das bandförmige Material mit einem ziehenden Schnitt abgeschnitten.

Eine Fehlfunktion bei der bekannten Vorrichtung entsteht dadurch, daß der Benutzer, nachdem er die Bandschneideeinrichtung in seine maximale Auslenkungsposition bewegt hat, die Vorschubeinrichtung betätigt. Da jedoch in diesem Fall das Messer der Bandschneideeinrichtung noch im Bandtransportweg liegt, verhindert das Messer den Bandtransport und es kann unterhalb des Schlitzes ein Bandstau entstehen, der die weitere Funktionsfähigkeit der Vorrichtung beeinträchtigt. Selbst wenn entsprechende Warnhinweise auf der Vorrichtung angegeben sind, stellen sich diese Art von Fehlfunktionen immer wieder ein, da diese Warnhinweise häufig übersehen werden.

Andere Fehlfunktionen werden dadurch verursacht, daß das bandförmige Material bei der Vorschubbewegung zurück in die vertikal darunterliegende Bandaufnahmeeinrichtung zurückfällt. Dies rührt daher, daß bei Betätigung der Bandvorschubeinrichtung diese für eine kurze Zeit das bandförmige Material nicht führt und festhält. Dann kann es passieren, daß das bandförmige Material aufgrund seiner Schwerkraft in die Bandaufnahmeeinrichtung zurückfällt und das bandförmige Material manuell neu in den Bandvorschubbereich eingeführt werden muß.

Eine weitere Fehlfunktion hat sich dadurch gezeigt, daß die Fixierung der Klemmeinrichtung bei Auslenkung der Schneideeinrichtung unzulänglich ist. Zwar wäre es denkbar, die Klemmeinrichtung zu verbreitern, um letztendlich den Schlitz bei der Auslenkung der Bandschneideeinrichtung zu verengen, jedoch führt dies zu unerwünschten Druckstellen am bandförmigen Material. Diese Druckstellen können dazu führen, daß ein entnommenes Wund- oder Heftpflaster aufgrund der Quetschung im Bereich der Klemmeinrichtung unbrauchbar ist.

Deshalb darf der Schlitz bei Auslenkung der Bandschneideeinrichtung nicht zu eng sein. Andererseits wiederum neigt das bandförmige Material beim Schneiden dazu, sich mit dem Messer der Bandschneideeinrichtung in dessen Richtung zu bewegen, wenn es abgeschnitten wird, weil der ziehende Schnitt eine relativ große Kraft quer zur Bandtransportrichtung auf das bandförmige Material ausübt.

Der Erfindung liegt daher die Aufgabe zugrunde, die bekannte Vorrichtung zu verbessern und insbesondere die erwähnten Probleme zu beseitigen und einen zuverlässigen Einsatz der Portioniervorrichtung zu ermöglichen.

Diese Aufgabe wird erfindungsgemäß bei einer Vorrichtung der eingangs beschriebenen Art dadurch gelöst, daß ein weiteres oder mehrere weitere Mittel zur Verhinderung eines unerwünschten Transportes oder einer unerwünschten Bewegung des bandförmigen Materials ausgebildet sind.

Ein solches erfindungsgemäßes Mittel verhindert die unzulässige Betätigung der Bandvorschubrichtung und/oder ein Verrutschen des bandförmigen Materials beim Schneiden und/oder ein Zurückfallen des bandförmigen Materials in die Bandaufnahmeeinrichtung bei Betätigung der Vorschubeinrichtung. Vorteilhafte Weiterbildungen und Ausführungen der Erfindung sind in den Unteransprüchen beschrieben.

Es ist zweckmäßig, das Mittel zur Verhinderung eines unerwünschten Bandtransportes durch die Bandschneideeinrichtung selbst auszubilden, welche vollständig oder teilweise bei Auslenkung in eine Öffnung in der Vorschubeinrichtung durchsetzt, so daß das die Öffnung durchsetzende Teil der Bandschneideeinrichtung im Bewegungsweg der Vorschubeinrichtung liegt und ihre Betätigung verhindert.

Dadurch wird insbesondere verhindert, daß nach dem Abschneiden eines Bandes die Betätigung der Vorschubeinrichtung erst dann wieder freigegeben wird, wenn die Bandschneideeinrichtung sich in ihrer Anfangsposition befindet. Da sich die Bandschneideeinrichtung nach dem Schneiden des Bandes in einer von der Anfangsposition größtmöglichen entfernten Endposition befindet, durchsetzt die Bandschneideeinrichtung bzw. ein Teil davon vorzugsweise nur in dieser Position die Öffnung der Vorschubeinrichtung.

Dies kann vorteilhafterweise dadurch realisiert werden, daß die Vorschubeinrichtung eine manuell betätigbare Taste mit einer Öffnung aufweist, die im Messerweg der Bandschneideeinrichtung liegt. Nimmt die Bandschneideeinrichtung ihre Endposition nach dem Abschneiden des Bandes ein, so liegt das Messer als Teil der Bandschneideeinrichtung in der Öffnung und verhindert ein Herunterdrücken der Taste, so daß kein weiterer Bandvorschub erfolgen kann. Erst nachdem die Bandschneideeinrichtung aus ihrer Endposition wieder wegbewegt ist, wird die Taste zur Betätigung der Bandvorschubeinrichtung freigegeben.

Es ist zweckmäßig, das Mittel zur Verhinderung einer unerwünschten Bandbewegung zusätzlich zu dem Vorbeschriebenen oder alternativ dazu durch ein Schlitzverengungsmittel auszubilden, welches einen Bandschlitz zwischen der Bandaufnahmeeinrichtung und der Vorschubeinrichtung so verengt, daß es eine unerwünschte Bandbewegung entgegen der Bandvorschubrichtung verhindert.

Durch die Ausbildung des Schlitzverengungsmittels wird ein Zurückfallen des bandförmigen Materials aufgrund seiner Schwerkraft vor allem dann verhindert, wenn die Bandvorschubeinrichtung bei ihrer Betätigung das Band weder berührt noch festhält.

Als ein einfaches Schlitzverengungsmittel werden Nocken und/oder Federelemente und/oder Wulste vorgeschlagen, die in einem Schlitz zwischen der Bandaufnahmeeinrichtung und der Bandvorschubrichtung liegen und einen oberen Rand aufweisen, der etwa senkrecht zum bandförmigen Material ausgerichtet ist oder zu diesem in einem stumpfen Winkel angeordnet ist. Das Schlitzverengungsmittel kann von dem bandförmigen Material in Bandtransportrichtung quasi widerstandsfrei passiert werden, während entgegen der Bandvorschubrichtung durch die Schlitzeinengung und durch die Randausbildung des Schlitzverengungsmittels ein Rückfall in die Bandaufnahme aufgrund der Schwerkraft verhindert wird.

Das Mittel zur Verhinderung unerwünschter Bandbewegungen kann zusätzlich oder alternativ zu den vorbeschriebenen Mitteln durch eine Oberflächenstrukturierung auf der vom bandförmigen Material zugewandten Seite der Klemmeinrichtung gebildet sein. Durch die Oberflächenstrukturierung weist die dem bandförmigen Material zugewandte Seite der Klemmeinrichtung eine ausreichende Rauhheit auf, so daß die Reibung zwischen der Klemmeinrichtung und dem bandförmigen Material beim Schneiden erhöht ist. Dadurch wird ein Verrutschen des bandförmigen Materials in Bandschneiderichtung wirksam verhindert.

Vorzugsweise ist die Oberflächenstrukturierung der Klemmeinrichtung durch Zähne gebildet, die am oberen Rand der Klemmeinrichtung parallel zur Bandvorschubrichtung und somit im wesentlichen senkrecht zur Bandschneiderichtung ausgerichtet sind. Es hat sich gezeigt, daß durch die Ausbildung der Zähne die bekannte effektive Schlitzbreite beibehalten werden kann und keine Beschädigungen am Band beim Schneiden durch die Zähne hervorgerufen werden.

Es sei ausdrücklich erwähnt, daß bereits jedes einzelne der vorbeschriebenen Mittel zur Verhinderung einer unerwünschten Bandbewegung die Funktionsweise der bekannten Vorrichtung erheblich verbessert, und daß durch die Ausbildung der Mittel zur Verhinderung der Betätigung der Bandvorschubeinrichtung bei Auslenkung der Bandschneideeinrichtung, der Schlitzverengungsmittel und der Oberflächenstrukturierungsmittel auf der Klemmeinrichtung zusammen eine Vorrichtung geschaffen wird, die sämtliche dem bekannten Stand der Technik anhaftenden Probleme beseitigt. Die Kombination aller Mittel verbessert die Funktionsweise erheblich und sorgt für ein zuverlässiges Abschneiden des bandförmigen Materials und gewährleistet eine gute Betriebsbereitschaft der erfindungsgemäßen Vorrichtung.

Ferner ist es zweckmäßig, daß die erfindungsgemäße Vorrichtung zur Lenkung der Taste der Vorschubseinrichtung erste und zweite Schlitze aufweist, die von Vorsprüngen der Taste durchsetzt werden. Zweckmäßigerweise beträgt der Winkel zwischen den ersten und zweiten Schlitzen etwa 90°. Dadurch wird eine ausreichende Fixierung und Führung der Taste erreicht.

Weiter ist es vorteilhaft, daß die Bandschneideeinrichtung ein auswechselbares Messer aufweist, welches von einer Halterung aufgenommen ist und das Messer wenigstens eine Öffnung aufweist, die von einem Eingriffsmittel, welches auf einem in die Halterung steckbaren Festlegungsmittel ausgebildet ist, durchsetzbar ist.

Außerdem ist es vorteilhaft, wenn zusätzlich oder alternativ zu den beschriebenen Ausbildungen die Vorrichtung eine Schutzabdeckung für den Ausgabeschlitz aufweist, und die Schlitzabdeckung dafür sorgt, daß, wenn die Vorrichtung sich nicht im Betriebseinsatz befindet, Verschmutzungen oder Verunreinigungen nicht in den Schlitz und an das darunterliegende bandförmige Material gelangen und dieses unbrauchbar machen bzw. kontaminieren können.

Nachfolgend wird die Erfindung anhand eines in Zeichnungen dargestellten Ausführungsbeispiels näher erläutert.

In der Zeichnung stellen dar:
- Fig. 1: eine Draufsicht auf eine Ausführungsform der Vorrichtung gemäß der Erfindung;
- Fig. 2: eine Frontansicht der in Fig. 1 dargestellten Vorrichtung;
- Fig. 3: eine Draufsicht auf den Führungsbereich der Bandschneideeinrichtung;
- Fig. 4: den unter der Bandschneideeinrichtung angeordneten Rahmen und die Vorschubeinrichtung in Draufsicht;
- Fig. 5: eine Schnittansicht des oberen Teils der Vorrichtung;
- Fig. 6: eine Schnittansicht entlang der Linie A-A in Fig. 4;
- Fig. 7a: den Messerhalter der Bandschneideeinrichtung;
- Fig. 7b: ein Festlegungselement für das Messer;
- Fig. 7c: ist eine Explosionsdarstellung der in Fig. 7a dargestellten Halterung;
- Fig. 8: eine weitere Schnittansicht entlang der Linie B-B in Fig. 2;
- Fig. 9 und 10: zwei alternative Ausbildungen des Schlitzverengungsmittels;
- Fig. 11: eine Draufsicht auf eine weitere Ausführungsform der Erfindung; und
- Fig. 12: eine Seitenansicht der in Fig. 11 dargestellten Ausführungsform.

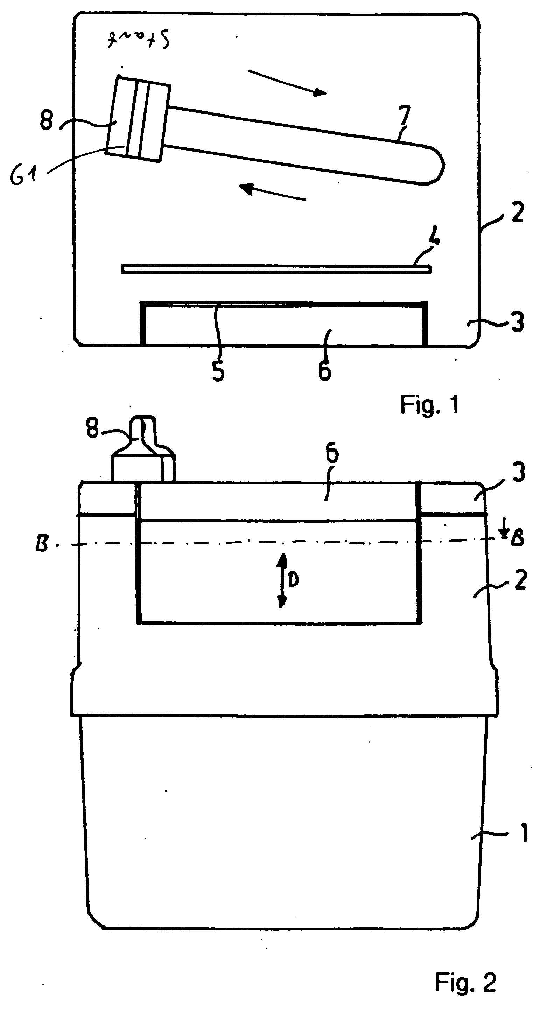

Fig. 1 und 2 zeigen eine Vorrichtung zur Portionierung von bandförmigem Material mit einer als Vorratsbehälter 1 ausgebildeten Bandaufnahmeeinrichtung, die den unteren Teil der Vorrichtung bildet. Der obere Teil 2 der Vorrichtung weist eine Bandschneideeinrichtung 61, eine Ausgabeöffnung 4 für das bandförmige Material und eine Taste 6 auf, die mit einer Bandvorschubeinrichtung innerhalb der Vorrichtung zusammenwirkt. die Bandschneideeinrichtung weist ein Griffstück 8 auf, welches sich in Fig. 1 in der Startposition befindet. Ferner ist der obere Teil 2 der Vorrichtung mit einer abklapp- oder abhebbaren Kappe 3, die die Öffnung 4 und eine Auslassung 7 für das Griffstück 8 aufweist, abgedeckt.

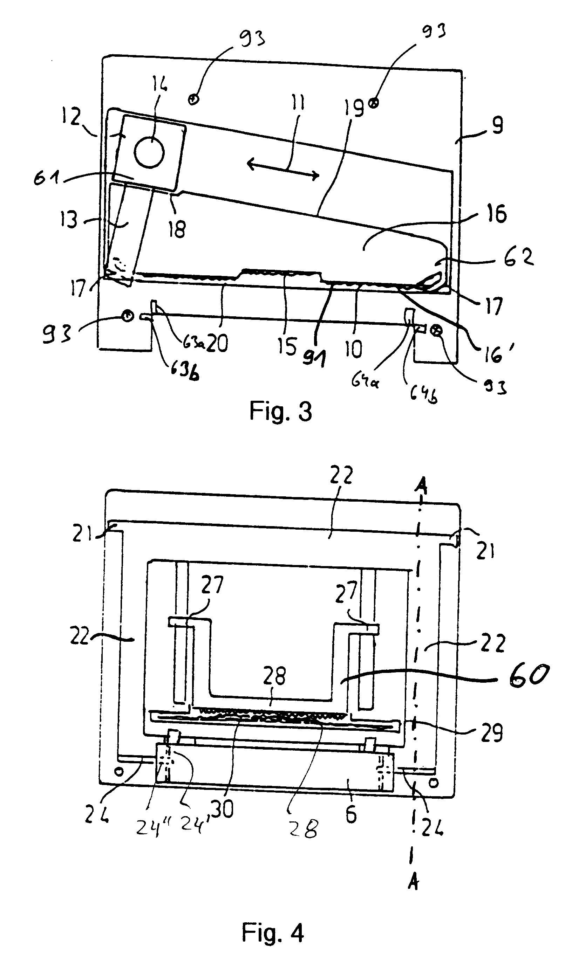

Wenn die Abdeckung 3 wie in Fig. 3 gezeigt abgenommen ist, liegt die Führungsplatte 9 für die Bandschneideeinrichtung 61 frei.

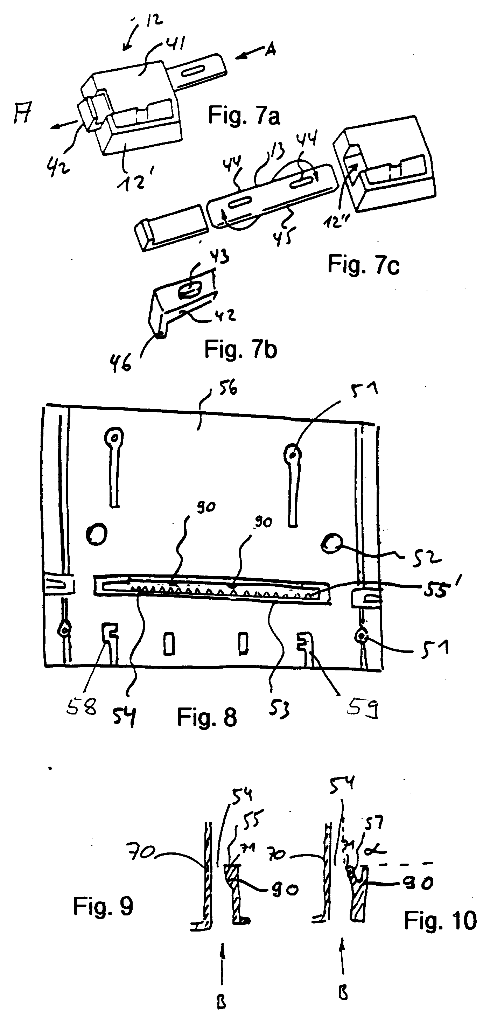

In der Führungsplatte 9 ist eine schräg im Winkel zum Schlitz 10 für die Pflasterbahn verlaufende, schwach konisch ausgebildete Nut 11 vorgesehen. Der Winkel, in dem diese Nut verläuft, entspricht dem Winkel der Auslassung 7 von Fig. 1. In dieser Nut 11 ist ein Gleitstück 12 vorgesehen, in das ein Messer 13 einsetzbar ist. Die Zusammensetzung des Gleitstücks und des Messers wird unter Bezugnahme auf Fig. 7 später beschrieben. Zentrisch im Gleitstück befindet sich eine kreisrunde Öffnung 14, die ebenfalls konisch ausgebildet sein kann und in die ein entsprechendes konisches Teil des Griffstücks 8 eingreift. Bei der Längsbewegung des Gleitstückes 12 innerhalb der Nut vollzieht das Messer 13 der Bandschneideeinrichtung einen ziehenden Schnitt längs des Schlitzes 10.

Der Schlitz 10 weist ferner in der Mitte eine Verbreiterung 15 auf, um zu ermöglichen, daß der die Wunde bedeckende Teil des Pflasters beim Transport durch den Schlitz nicht verklemmt bzw. gequetscht wird.

Um nun einen sauberen Schlitz zu erhalten, wird die Pflasterbahn während des Schnittes im Schlitz 10 festgeklemmt. Zu diesem Zweck ist der Teil 16 zwischen dem Schlitz und der Nut 11 als bewegliche Klemmeinrichtung ausgebildet und an der dem Schlitz zugewandten Seite mit Federelementen 17 versehen. Die Federelemente können Schraubenfedern oder, wie dargestellt, fingerartige elastische Vorsprünge sein, die einstückig mit der Klemmeinrichtung 62 verbunden sind. Im Ruhezustand befindet sich das Gleitstück 12 innerhalb einer Aussparung 18 an der dem Schlitz abgewandten Seite der Klemmeinrichtung 62. Wenn das Gleitstück 12 ausgelenkt wird, läuft es auf eine höher liegende Kante 19 des Teiles 16 auf und drückt dieses als Klemmkeil unter den Druck der Federelemente 17 zur Verengung des Schlitzes 10 gegen eine feste Wand 20 des Schlitzes. Dadurch wird das sich im Schlitz 10 befindliche Bandstück eingeklemmt und das Messer kann im ziehenden Schnitt das Abschneiden bewirken.

Zur Erhöhung der Rutschfestigkeit beim Schneiden weist die dem Schlitz 10 zugewandte Seite der Klemmeinrichtung 62 bzw. des Teiles 16 eine Oberflächenstrukturierung auf, durch die die Oberflächenrauhheit des Klemmkeils erhöht ist. Die Oberflächenstrukturierung der Klemmeinrichtung ist durch Zähne 16' gebildet, die am oberen Rand des Klemmblocks 16 parallel zur Bandvorschubrichtung ausgerichtet liegen.

Das Teil 16 der Klemmeinrichtung weist an seiner dem Schlitz 10 zugewandten Seite in der Mitte eine Verbreiterung 15 auf, dessen Weite in etwa dem Inneren, des auf der Wunde aufliegenden Mullteils eines Pflasterverbandes entspricht. Je nachdem welche Art von Pflastermaterial verwendet wird, kann das Teil 16 gegen ein anderes Teil 16 mit einer anderen Verbreiterungsweite ausgetauscht werden, so daß eine entsprechende Anpassung des Teils 16 an die Breite des Mullteils von Pflastermaterial vorgenommen werden kann. Die Anpassung der Verbreiterung 15 des Teils 16 der Klemmeinrichtung an die Bandbreite bzw. die Breite des Mullteils eines Pflasterverbandes hat Vorteile beim Schneiden und vermeidet, daß das Mullteil beim Schneiden unnötigerweise gequetscht wird.

Die Führungsplatte 9 weist Schlitze 63a und 63b sowie 64a und 64b auf, die in Draufsicht betrachtet senkrecht zueinander angeordnet liegen. Die Schlitze werden von entsprechenden Vorsprüngen 24' und 24'' der Taste 6 durchsetzt, so daß die Taste 6 nur in vertikaler Richtung bewegbar ist.

In den Fig. 4, 5 und 6 ist die Vorschubeinrichtung für das abzuschneidende bandförmige Material dargestellt. Demgemäß befindet sich unterhalb der Führungsplatte 9 ein an zwei Achsen 21 in der Gehäusewand des Oberteils 2 schwenkbar gelagerter Rahmen 22. Dieser Rahmen 22 ist mittels einer vor dem Schlitz 4 bzw. 10 senkrecht beweglich geführten Taste 6 gegen den Druck von Federn 23, die unterhalb des Rahmens 22 anliegen, schwenkbar. Der Rahmen 22 weist hierzu zwei Vorsprünge 24 auf, die in entsprechende Aussparungen 25 innerhalb der Taste eingreifen.

Alternativ dazu kann der Rahmen mittels der auf das bandförmige Material gerichteten Vorsprünge 25' der Taste 6, die über den Vorsprüngen des Rahmens zur Anlage kommen, heruntergedrückt werden. Die Vorsprünge 24' durchsetzen gleichzeitig dabei die entsprechenden Schlitze 63a und 64a und die Vorsprünge 24'' der Taste 6 durchsetzen die Aussparungen 63b und 64b.

Im Inneren des Rahmens 22 ist ein in zwei Längsschlitzen 26 mit Achsen 27 geführter Mitnehmer 28 angeordnet, der beim Herunterdrücken der Taste 6 freiliegt und bei der Aufwärtsbewegung der Taste 6 gegen die in der Führung 29 verlaufende Materialbahn angelegt wird und sie entsprechend der Aufwärtsbewegung der Taste um ein Stück nach oben aus den Schlitzen 10 bzw. 4 herausschiebt. Der Mitnehmer weist im vorderen Bereich, der auf das bandförmige Material gerichtet ist, Zähne 28' auf, um ein sicheres Erfassen des bandförmigen Materials zu gewährleisten.

Außerdem sind im Inneren des Oberteils 2 je zwei Kurvenbahnen 31 und 32 vorgesehen, die an den Achsen 27 anliegen und sie entsprechend der Auf- bzw. Abwärtsbewegung führen, damit der oben beschriebene Effekt eintritt.

Selbstverständlich kann der beschriebene Nachschubmechanismus durch eine andere Einrichtung, z.B. durch eine Walze oder durch andere Hebelmechanismen ausgebildet werden. Schließlich kann anstelle des Vorratsbehälters 1 auch eine Rollen- oder Walzenanordnung oder eine andere Ausbildung der Bandaufnahmeeinrichtung vorgesehen werden.

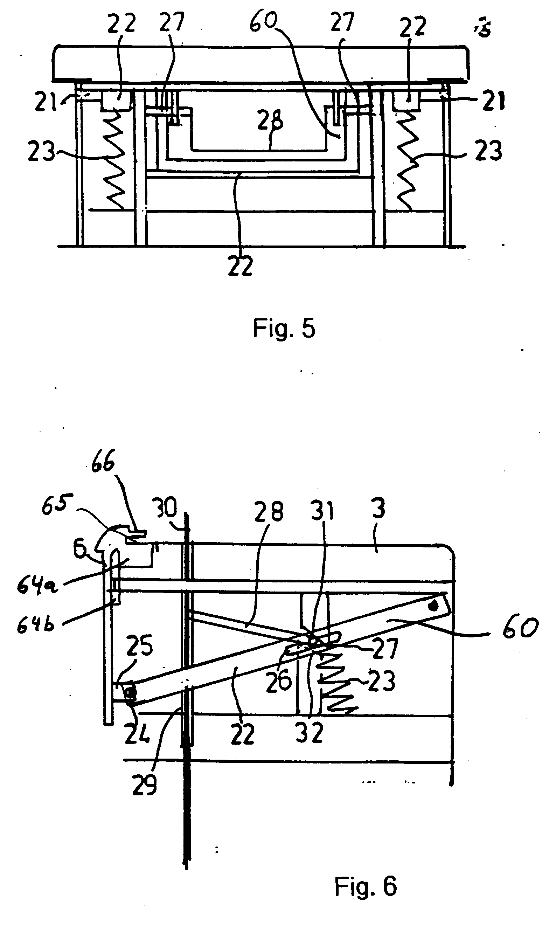

Wie in Fig. 6 oben links zu sehen, weist die Taste 6 in ihrem oberen Bereich eine Öffnung 65 auf, die durch eine Kerbe zwischen dem auf die Materialbahn zu weisenden Vorsprung 64a und einem darüber angeordneten Vorsprung 66 gebildet ist. Die Kerbe liegt, wenn die Tast nicht betätigt wird, genau in der Schnittlinie des Messers der Bandschneideeinrichtung und befindet sich dort, wo das Messer der Bandschneideeinrichtung nach dem Schneiden zur Anlage kommt. Dann durchsetzt das Messer die Kerbe 65 und verhindert, daß die Taste betätigt werden kann. Somit kann es nicht passieren, daß die Bandvorschubeinrichtung betätigt wird, wenn die Bandschneideeinrichtung sich in der Endposition befindet.

Es ist ebenso möglich, den Vorsprung parallel zum oberen Rand der Taste und parallel zur Schnittebene weiterzuführen, so daß das Messer grundsätzlich bereits bei einer geringen Auslenkung der Bandschneideeinrichtung die durch die Kerbe gebildete Öffnung durchsetzt.

Fig. 7 zeigt die als Gleitstück 12 ausgebildete Messerhalterung 12', welche ein durch die Halterung 12' durchgehendes Loch 12'' aufweist, in welches das Messer 45 und ein Festlegungsmittel 42 für das Messer einsteckbar sind. Das Messer weist längliche Schlitze oder Aussparungen 44 auf, in die ein Vorsprung 43 des Festlegungsmittels 42 als Eingriffsmittel steckbar ist. Das Festlegungsmittel weist eine abstehende Kante 46 auf, so daß das Festlegungsmittel nicht durch das Loch 12'' gesteckt werden kann, sondern nur bis zu einer gewissen Tiefe in das Loch. Gleichzeitig kann das Festlegungsmittel eine Verkeilung des Messers in dem Gleitstück 12 bewirken, so daß das Messer darin festliegt und sich nicht aus der Halterung lösen kann.

Wie in Fig. 7c dargestellt, weist das Messer zwei hintereinander ausgebildete längliche Bohrungen 44 auf, so daß das Messer, wenn eine Seite verschlissen bzw. abgestumpft ist, zusammen mit dem Festlegungsmittel aus der Halterung herausgenommen werden kann und dann umgedreht wird, so daß dann der noch scharfe Messerbereich, der vorher innerhalb der Halterung lag, nach außen steht.

Wie in Fig. 7a zu sehen, ist das Messer zusammen mit dem Festlegungsmittel 42 in Pfeilrichtung A aus dem Gleitstück 12 bewegbar.

In Fig. 8 ist ein Schnittbild entlang der Linie B-B in Fig. 2. Die Taste 6 und die bewegbaren Teile der Vorschubeinrichtung sind in der Darstellung weggelassen. Der mittlere Teil 2 weist Führungsschlitze 58 und 59 auf, welche in der Flucht der entsprechenden Schlitze 63b und 64b liegen. Durch einen Schlitz 54 gelangt das Bandmaterial in den Bereich der Vorschubeinrichtung. Das Bezugszeichen 52 deutet die Halterung für die in Fig. 5 und 6 dargestellten Federn an. Die Aufnahmen 51 sind zur Aufnahme von Schrauben vorgesehen, mit der der obere Teil 3 mit dem mittleren Teil 2 befestigt wird. Die Aufnahmen 51 sind in Verlängerung der in Fig. 3 dargestellten Schraubenaufnahmen 93 angeordnet.

Außerdem weist der Schlitz 54 Schlitzverengungsmittel 90 auf, welche einen in Fig. 9 und 10 dargestellten Querschnitt aufweisen können. 56 ist eine Basisplatte des mittleren Teils 2 zwischen der Bandaufnahmeeinrichtung und der Vorschubeinrichtung. Darüber hinaus weist die dem Schlitzverengungsmittel gegenüberliegende Wand eine in Bandvorschubrichtung ausgerichtete Oberflächenstrukturierung 55' auf, die eine Verkantung und ein seitliches Verrutschen des bandförmigen Materials verhindert.

Wie in den Fig. 9 und 10 dargestellt, wird der Schlitz 54 durch eine gerade Schlitzwand 70 und einen schlitzverengenden Nocken 55 gebildet. Der Nocken 55 leistet dem Bandmaterial in Zuführrichtung - Pfeil B - kaum einen Widerstand, weil er die Schlitzweite kontinuierlich bis auf ein vorgegebenes Maß verringert. Dagegen weist der Nocken 55 an seiner Oberseite einen Rand 71 auf, der senkrecht bzw. rechtwinklig zur Bandtransportrichtung liegt. Bandmaterial, welches dazu neigt aufgrund der Schwerkraft in die Bandaufnahmeeinrichtung aufgrund der Schwerkraft zurückzugleiten, wird von dem Rand 71 des Nockens 55 erfaßt, so daß ein unerwünschtes Zurückgleiten in die Bandaufnahmeeinrichtung verhindert wird.

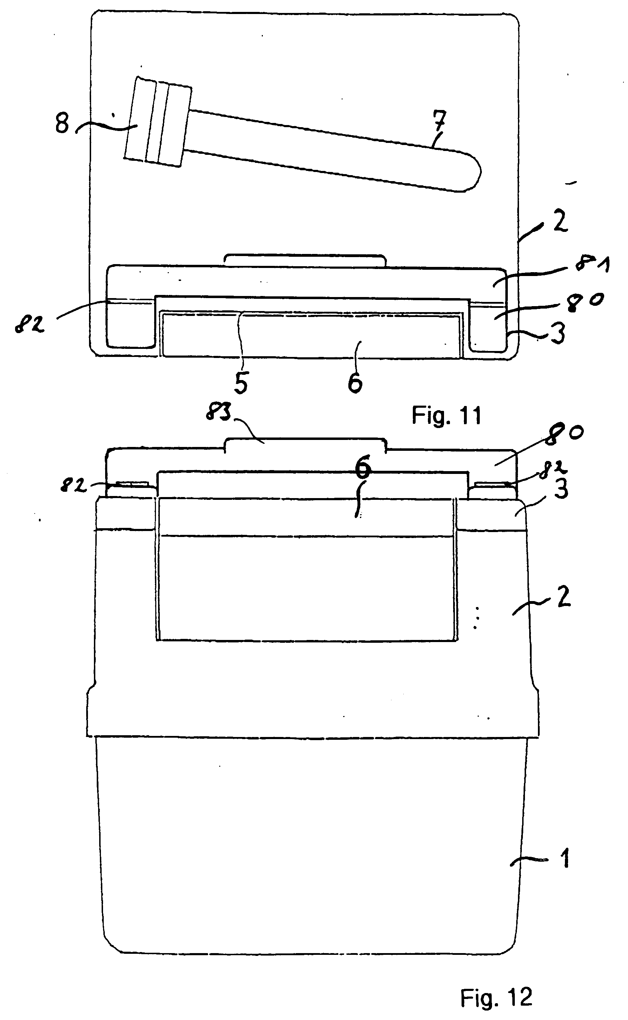

Fig. 10 zeigt eine zu Fig. 9 alternative Ausführung einer Schlitzverengung, die durch ein Y-förmiges Federelement 57 gebildet ist. Ein Schenkel des Y-förmigen Federelements aus flexiblem Material ist in Richtung auf die Schlitzwand gerichtet, so daß sich der Schlitz in Bandtransportrichtung kontinuierlich verengt. Der obere Rand des auf die Schlitzwand gerichteten Schenkels weist einen entgegen der Bandzuführrichtung gerichteten Richtung Winkel α dazu auf, der statt 90° auch im Bereich zwischen 75 und 175° liegen kann. Fig. 11 und 12 zeigen eine weitere vorteilhafte Ausführungsform der erfindungsgemäßen Vorrichtung. Dabei wird der Ausgabeschlitz 4 der Vorrichtung durch eine wegklappbare Schutzabdeckung 80 bedeckt. Die Abdeckung 80 ist am oberen Teil 3 der Vorrichtung befestigt oder einstückig mit ihr ausgebildet. Ferner weist die Abdeckung 80 Scharniere 82 auf, an denen der wegklappbare Teil 81 der Abdeckung 80 befestigt ist. Die Klappe 81 weist darüber hinaus einen Griff 83 auf. Die Klappe kann manuell in eine vertikal ausgerichtete Position gebracht werden, damit der Ausgabeschlitz freigelegt ist. Durch Betätigung der Bandvorschubrichtung und durch Vorschub des Bandes kann jedoch ebenfalls die Klappe nach oben weggeschoben werden, so daß für den Fall, daß der Benutzer das Wegklappen der Schutzabdeckung vergißt, trotzdem eine Ausgabe des bandförmigen Materials erfolgen kann.

Aus der vorhergehenden Beschreibung wird deutlich, daß die Mittel zur Führung der Taste und/oder Ausbildung der Messeraufnahme der Bandschneideeinrichtung und/oder Ausbildung der Schutzabdeckung unabhängig von den Mitteln zur Verhinderung einer unerwünschten Bewegung des bandförmigen Materials ausgebildet sein können und die Offenbarung der Anmeldung nicht so verstanden werden darf, daß diese Ausbildungen grundsätzlich zusammen mit den Mitteln zum Verhindern der unerwünschten Bandbewegung angeordnet sein müssen. Auch ohne die Mittel zur Verhinderung einer unerwünschten Bandbewegung erfüllt das Mittel zur Führung der Taste 6 und/oder die beschriebene Messeraufnahme der Bandschneideeinrichtung 61 und/oder die Schutzabdeckung 80 eine wie oben beschriebene vorteilhafte Funktion und sorgt für eine Verbesserung der bekannten Vorrichtung nach G 92 00 572.1. Die Kombination aller verschiedenen Merkmale ist jedoch problemlos möglich und sorgt für einen überaus zuverlässigen Betrieb der beschriebenen Vorrichtung zur Portionierung von bandförmigem Material 30.

## Patentansprüche

1. Vorrichtung zur Portionierung von bandförmigem Material (30), insbesondere Wund- oder Heftpflaster, mit einer Einrichtung (60) zum Vorschub des bandförmigen Materials von einer Bandaufnahmeeinrichtung (1), einer auslenkbaren Bandschneideeinrichtung (61) und einer Klemmeinrichtung (62), die das bandförmige Material bei Auslenkung der Bandschneideeinrichtung (61) im wesentlichen fixiert,
dadurch gekennzeichnet, daß wenigstens ein weiteres Mittel (62, 90, 16') zur Verhinderung eines unerwünschten Transportes oder einer unerwünschten Bewegung des bandförmigen Materials ausgebildet ist.

2. Vorrichtung nach Anspruch 1,
dadurch gekennzeichnet, daß das Mittel durch die Bandschneideeinrichtung (62) gebildet ist, welche bei Auslenkung mit einem Teil (13) im Bewegungsweg (D) eines Teils der Vorschubeinrichtung liegt und die Betätigung der Vorschubeinrichtung (60) eines Teils (6) verhindert.

3. Vorrichtung nach Anspruch 1,
dadurch gekennzeichnet, daß das Mittel durch ein Schlitzverengungsmittel (90) gebildet ist, das einen Bandschlitz (54) zwischen der Bandaufnahmeeinrichtung (1) und der Vorschubeinrichtung (60) so verengt, daß es eine unerwünschte Bandbewegung entgegen der Bandvorschubrichtung (B) verhindert.

4. Vorrichtung nach Anspruch 1,
dadurch gekennzeichnet, daß das Mittel durch eine Oberflächenstrukturierung (91) auf der vom bandförmigen Material (30) zugewandten Seite der Klemmeinrichtung (62) gebildet ist und die Oberflächenstrukturierung (91) ein unerwünschtes Verrutschen des bandförmigen Materials (30) beim Schneiden verhindert.

5. Vorrichtung nach Anspruch 2,
dadurch gekennzeichnet, daß die Vorschubeinrichtung (60) eine Taste (6) mit einer Öffnung (6') aufweist, daß ein Teil (13) der ausgelenkten Bandschneideeinrichtung die Öffnung (6') der Taste (6) so durchsetzt, daß das die Öffnung (61) durchsetzende Teil (13) im Bewegungsweg (D) der Taste (6) liegt.

6. Vorrichtung nach Anspruch 5,
dadurch gekennzeichnet, daß das Messer (13) der Bandschneideeinrichtung (61) in der als Einkerbung ausgebildeten Öffnung (6') der Taste (6) zur Anlage kommt, wenn die Bandschneideeinrichtung (61) ihre maximale Auslenkungsposition einnimmt.

7. Vorrichtung nach Anspruch 3,
dadurch gekennzeichnet, daß das Schlitzverengungsmittel (90) durch einen Nocken (55) und/oder durch ein Federelement (57) und/oder durch eine Wulst gebildet ist, und daß das Schlitzverengungsmittel (90) einen Rand (71) aufweist, der entgegen der Bandvorschubrichtung (B) in einem Winkel (α) von etwa 75 bis 170°, vorzugsweise 90° ausgerichtet ist.

8. Vorrichtung nach Anspruch 4,
dadurch gekennzeichnet, daß die Oberflächenstrukturierung (91) der Klemmeinrichtung (62) durch Zähne (16') gebildet ist, die am oberen Rand der Klemmeinrichtung (62) auf der dem bandförmigen Material (30) zugewandten Seite liegen.

9. Vorrichtung nach Anspruch 8,
dadurch gekennzeichnet, daß die Zähne (16') parallel zur Bandvorschubrichtung (B) ausgerichtet sind und bei Auslenkung der Bandschneideeinrichtung (61) eine Rutschbewegung des bandförmigen Materials in Auslenkrichtung (11) der Bandschneideeinrichtung verhindern.

10. Vorrichtung nach einem der vorhergehenden Ansprüche,
dadurch gekennzeichnet, daß die Vorrichtung im Bereich der Taste (6) erste und zweite Schlitze (63, 64) aufweist, und daß die Taste Vorsprünge (64) aufweist, die die Schlitze (63, 64) durchsetzen.

11. Vorrichtung nach Anspruch 10,
dadurch gekennzeichnet, daß der Winkel zwischen dem ersten und zweiten Schlitz etwa 90° beträgt.

12. Vorrichtung nach einem der vorhergehenden Ansprüche,
dadurch gekennzeichnet, daß die Bandschneideeinrichtung (61) ein Messer (13) aufweist, welches von einer Halterung aufgenommen ist, und das Messer (13) wenigstens eine Öffnung (44) aufweist, die von einem Eingriffsmittel (43) durchsetzbar ist und das Eingriffsmittel (43) auf einem in die Halterung steckbaren Festlegungsmittel (42) ausgebildet ist.

13. Vorrichtung nach einem der vorhergehenden Ansprüche,
gekennzeichnet durch eine wegbewegbare Abdeckung (80) zum Schutz der Ausgabeöffnung (4).

## Claims

1. Apparatus for dispensing ribbon-like material (30), in particular adhesive plaster, having a device (60) for advancing the ribbon-like material from a ribbon receptacle (1), a displaceable ribbon-cutting device (61) and a clamping device (62), which substantially fixes the ribbon-like material upon displacement of the ribbon-cutting device (61), characterised in that at least one further means (62, 90, 16') is provided for preventing undesired transport or undesired movement of the ribbon-like material.

2. Apparatus according to Claim 1, characterised in that the means is formed by the ribbon-cutting device (62) [sic], which, upon displacement, lies with a part (13) in the movement path (D) of a part of the advancing device and prevents the actuation of the advancing device (60) of a part (6).

3. Apparatus according to Claim 1, characterised in that the means is formed by a slot-narrowing means (90), which narrows a ribbon slot (54) between the ribbon receptacle (1) and the advancing device (60) in such a way that it prevents undesired ribbon movement counter to the ribbon-advancing direction (B).

4. Apparatus according to Claim 1, characterised in that the means is formed by surface structuring (91) on that side of the clamping device (62) which faces the ribbon-like material (30) and the surface structuring (91) prevents undesired slipping of the ribbon-like material (30) as it is being cut.

5. Apparatus according to Claim 2, characterised in that the advancing device (60) has a push-button (6) with an opening (6'), in that a part (13) of the displaced ribbon-cutting device passes through the opening (6') of the push-button (6) in such a way that the part (13) passing through the opening (61) [sic] lies in the movement path (D) of the push-button (6).

6. Apparatus according to Claim 5, characterised in that the cutter (13) of the ribbon-cutting device (61) comes to rest in the opening (6'), constructed as a notch, of the push-button (6) when the ribbon-cutting device (61) assumes its position of maximum displacement.

7. Apparatus according to Claim 3, characterised in that the slot-narrowing means (90) is formed by a lug (55) and/or by a spring element (57) and/or by a bulge, and in that the slot-narrowing means (90) has an edge (71) which is oriented at an angle (α) of about 75 to 170°, preferably 90°, counter to the ribbon-advancing direction (B).

8. Apparatus according to Claim 4, characterised in that the surface structuring (91) of the clamping device (62) is formed by teeth (16') which lie at the upper edge of the clamping device (62) on the side facing the ribbon-like material (30).

9. Apparatus according to Claim 8, characterised in that the teeth (16') are oriented parallel to the ribbon-advancing direction (B) and, upon deflection of the ribbon-cutting device (61), prevent a slipping movement of the ribbon-like material in the displacement direction (11) of the ribbon-cutting device.

10. Apparatus according to one of the preceding claims, characterised in that the apparatus has first and second slots (63, 64) in the region of the push-button (6), and in that the push-button has projections (64) [sic] which pass through the slots (63, 64).

11. Apparatus according to Claim 10, characterised in that the angle between the first and second slot is about 90°.

12. Apparatus according to one of the preceding claims, characterised in that the ribbon-cutting device (61) has a cutter (13) which is received by a holder, and the cutter (13) has at least one opening (44), through which an engaging means (43) is able to pass, and the engaging means (43) is formed on a fastening means (42) capable of being pushed into the holder.

13. Apparatus according to one of the preceding claims, characterised by a cover (80) which can be moved away and serves to protect the dispensing opening (4).

## Revendications

1. Installation de distribution par tronçon d'une matière en bande (30), notamment des pansements adhésifs ou des sparadraps, comportant un dispositif (60) destiné à faire avancer de la matière en bande d'un dispositif (1) de réception de bande, un dispositif (61) de sectionnement de bande, qui peut être écarté, et un dispositif de serrage (62), qui immobilise sensiblement la matière en bande lorsque le dispositif (61) de sectionnement de bande est écarté,
caractérisé par le fait qu'au moins un autre moyen (62, 90, 16') est conformé pour empêcher tout acheminement non souhaité ou tout mouvement inopportun de la matière en bande.

2. Installation selon la revendication 1,
caractérisé par le fait que le moyen est constitué par le dispositif (62) de sectionnement de bande qui, lors de son écartement, se trouve par une partie (13) dans le trajet de déplacement (D) d'une partie du dispositif d'avancement et empêche l'actionnement du dispositif (60) d'avancement d'une partie (6).

3. Installation selon la revendication 1,
caractérisé par le fait que le moyen est constitué par un moyen (90) de rétrécissement de fente, qui rétrécit une fente (54) de passage de bande entre le dispositif (1) de réception de bande et le dispositif (60) d'avancement, de manière à empêcher tout mouvement non souhaité de la bande dans le sens (B) opposé à celui du dispositif d'avancement de bande.

4. Installation selon la revendication 1,
caractérisé par le fait que le moyen est constitué par une structuration de surface (91) du côté, tourné vers la matière en bande (30), du dispositif (62) de serrage et que la structuration de surface (91) empêche tout glissement inopportun de la matière en bande (30) lors du sectionnement.

5. Installation selon la revendication 2,
caractérisé par le fait que le dispositif (60) d'avancement présente une touche (6) comportant une ouverture (6'), qu'une partie (13) du dispositif de sectionnement de bande écarté passe dans l'ouverture (6') de la touche (6) de telle manière que la partie (13) passant dans l'ouverture (61) se trouve dans le trajet de déplacement (D) de la touche (6).

6. Installation selon la revendication 5,
caractérisé par le fait que la lame (13) du dispositif (61) de sectionnement de bande vient en butée dans l'ouverture (6'), en forme d'encoche, de la touche (6) lorsque le dispositif (61) de sectionnement de bande prend sa position d'écartement maximal.

7. Installation selon la revendication 3,
caractérisé par le fait que le moyen (90) de rétrécissement de fente est formé par une saillie (55) et/ou par un élément à ressort (57) et/ou par un bourrelet, et que le moyen (90) de rétrécissement de fente présente un bord (71) qui est orienté en formant un angle (α) d'environ 75 à 170°, et de préférence de 90°, dans le sens opposé à celui d'avancement (B) de la bande.

8. Installation selon la revendication 4,
caractérisé par le fait que la structuration de surface (91) du dispositif (62) de serrage est constituée par des dents (16'), qui sont situées sur le bord supérieur du dispositif (62) de serrage, du côté tourné vers la matière en bande (30).

9. Installation selon la revendication 8,
caractérisé par le fait que les dents (16') sont orientées parallèlement à la direction d'avancement (B) de la bande et empêchent, lors de l'écartement du dispositif de sectionnement de bande (61), un mouvement de glissement de la matière en bande dans le sens d'écartement (11) du dispositif de sectionnement de bande.

10. Installation selon l'une des revendications précédentes,
caractérisé par le fait que le dispositif comporte des première et deuxième fentes (63, 64) dans la zone de la touche (6), et que la touche présente des protubérances (64) qui passent dans les fentes (63, 64).

11. Installation selon la revendication 10,
caractérisé par le fait que l'angle entre les première et deuxième fentes est d'approximativement 90°.

12. Installation selon l'une des revendications précédentes,
caractérisé par le fait que le dispositif (61) de sectionnement de bande comporte une lame (13) qui est reçue par un élément de support, la lame (13) ayant au moins une ouverture (44) dans laquelle peut passer un moyen d'engagement (43), le moyen d'engagement (43) étant formé sur un moyen de fixation (42) enfichable dans l'élément de support.

13. Installation selon l'une des revendications précédentes,
caractérisé par un couvercle (80) amovible servant à protéger l'ouverture (4) de distribution.
